# EUROPÄISCHE PATENTSCHRIFT

(11) **EP 0 927 537 B1**
(45) Veröffentlichungstag und Bekanntmachung des Hinweises auf die Patenterteilung: **06.04.2005**
(21) Anmeldenummer: 98119485.5
(22) Anmeldetag: 15.10.1998
(51) Int. Cl.: A61B 5/103

(54) **Vorrichtung zur Lieferung eines akustischen Signals bei der Erfassung des auf einem Fuss lastenden Körpergewichts**
Means for delivering of an acoustic signal by sensing the load charged on a foot by the bodyweight
Dispositif pour délivrer un signal acoustique en détectant le chargement d'un pied par le poids du corps

(30) Priorität: 21.10.1997 DE 29718680 U
(43) Veröffentlichungstag der Anmeldung: 07.07.1999
(73) Patentinhaber: Sanostep Gesellschaft für Innovative Gesundheitstechnik mbH, 82436 Tauting (DE)
(72) Erfinder: Bechmann, Peter, 82487 Oberammergau (DE)

(56) Entgegenhaltungen:
- DE-A- 4 100 834
- US-A- 3 791 375
- US-A- 4 990 731

## Beschreibung

Die Erfindung bezieht sich auf eine Vorrichtung zur Lieferung eines akustischen Signals bei der Erfassung des auf einem Fuß lastenden Körpergewichts in Abhängigkeit von der Druckbelastung eines mit einem Fluid gefüllten, elastisch deformierbaren Fußkissens, das als ein Drucksensor für das Körpergewicht über eine Fluidleitung mit einem Druckwächter verbunden ist, der zur Betätigung eines Signalgebers in Abhängigkeit von dem über die Fluidleitung vermittelten Fluiddruck vorgesehen ist.

Vorrichtungen der vorgenannten Art dienen dem Zweck, bei der Therapie von Beinfrakturen sowie nach orthopädischen Operationen bspw. der Hüfte die Belastung des Fußes mit einem allmählich fortschreitenden Anteil des Körpergewichts nach bestimmten Vorgaben für den Patienten zu überwachen. So wird bspw. für die Therapie von einfacheren Beinfrakturen eine anfängliche Fußbelastung mit nur etwa 20 kg zugestanden, also nur mit einem Minimalanteil des Körpergewichts eines Erwachsenen, um bei den ersten Gehversuchen des Patienten nach einer Operation ein schlechtes Zusammenwachsen der Knochen zu verhindern. Die Lieferung eines akustischen Signals bei solchen Vorrichtungen soll in diesem Fall den Patienten folglich veranlassen, den Fuß wieder zu entlasten, um den Heilungsprozeß nicht unnötig zu verzögern.

Bei einer aus der US-A-3 974 491 bekannten Vorrichtung der vorgenannten Art ist das Fußkissen mit einer aus Schaumstoff bestehenden Innensohle des Schuhs ausgebildet, in welchen ein elastischer Schlauch mit einem schlangenlinienförmigen Verlauf im Fußballenbereich und mit einem schneckenförmigen Verlauf im Fersenbereich eingebettet ist. Der an seinem Ende verschlossene Schlauch ist mit Wasser gefüllt und ist über einen Verbindungsschlauch an eine elastisch deformierbare Blase angeschlossen, die als Druckwächter benutzt wird und dafür zur Befestigung bspw. mittels eines Verschlußbandes am Bein des Patienten vorgesehen ist. Diese Blase wirkt mit einem federbelasteten Betätigungshebel für einen Druckknopfschalter zusammen, sodaß in Abhängigkeit von dem an die Blase vermittelten und von diesem Betätigungshebel abgetasteten Fluiddruck der Druckknopfschalter für das Auslösen eines elektrischen Summers betätigt wird, sobald die mit dem Fußkissen als Drucksensor erfaßte Belastungsgrenze erreicht ist und dann mit einem Summton signalisiert wird. Bei dieser bekannten Vorrichtung ist nachteilig, daß die elektrische Ausführungsform des Signalgebers die Verwendung einer Batterie für den Betrieb des Summers voraussetzt und damit auch noch ein besonderer Betätigungsschalter benötigt wird, der zwischen den Druckwächter und den als Signalgeber fungierenden Summer zwischengeschaltet ist, wodurch die gesamte Vorrichtung entsprechend aufwendig ausgeführt ist und gleichzeitig auch einer erhöhten Störanfälligkeit unterliegt.

Der Erfindung liegt daher die Aufgabe zugrunde, eine Vorrichtung der eingangs genannten Art bereitzustellen, bei welchem das beim Erreichen einer vorgegebenen Belastungsgrenze vermittelte akustische Signal mit einfacheren und zuverlässigeren Maßnahmen geliefert werden kann.

Diese Aufgabe wird bei einer Vorrichtung der eingangs genannten Art dadurch erfindungsgemäß gelöst, daß der Signalgeber mit einer durch den Druckwächter zur Abgabe eines hörbaren Klickgeräusches betätigbaren Klickfeder ausgebildet ist.

Mit der erfindungsgemäß vorgesehenen Verwendung einer Klickfeder als Signalgeber wird somit eine rein mechanische Ausführungsform der Vorrichtung erhalten, bei welcher das beim Erreichen einer vorbestimmten Belastungsgrenze vermittelte und in bekannter Weise dann mit der Rückformung einer Auswölbung einer solchen Klickfeder erhaltene Klickgeräusch so laut hörbar ist, daß damit der Patient zu einer sofortigen Gewichtsentlastung des Beins veranlaßt wird, das sich nach einer Beinfraktur in einem Heilprozeß befindet. Weil sich bei dieser Gewichtsentlastung das hörbare Klickgeräusch wiederholt, ist damit für den Patienten auch die Überprüfung gesichert, daß er dann wieder davon weiß, daß er das Bein jetzt nicht mehr zu hoch belastet, bzw. es ist mit der erfindungsgemäßen Vorrichtung auch gewährleistet, daß solange das Klickgeräusch wiederholt geliefert wird, der Patient sich allmählich auf die zugestandene Belastungsgrenze für das Bein einpendeln wird, weil mit einer solchen wiederholten Verdoppelung des Klickgeräusches eine entsprechende Alarmbereitschaft des Patienten ausgelöst wird.

Weitere vorteilhafte und zweckmäßige Ausbildungen der erfindungsgemäßen Vorrichtung, die vorrangig ebenfalls nur eine rein mechanische Ausführungsform des Signalgebers beinhalten, sind in den einzelnen Ansprüchen angegeben. Die mechanische Ausführungsform des Signalgebers erlaubt dabei gleichzeitig die ebenfalls beanspruchte Bereitstellung eines autonomen, separat auswechselbaren Körpers für das Fußkissen und den Druckwächter, beide in der Ausbildung einer Blase aus einem elastisch deformierbaren Material und verbunden durch ein Schlauchstück, sodaß mit einer vorhergehenden Füllung dieses Körpers mit einem Fluid auch eine entsprechend problemlose Verwendungsmöglichkeit der Vorrichtung gesichert ist.

Ein Ausführungsbeispiel der Vorrichtung ist in der Zeichnung schematisch dargestellt und wird nachfolgend näher beschrieben. Es zeigt
- Fig. 1: eine Seitenansicht eines Beins mit einer Schemadarstellung der Gesamtheit der Vorrichtung,
- Fig. 2: eine Schnittansicht nach der Linie IV-IV in Fig. 4 der Vorrichtung im belastungsfreien Zustand ihres Drucksensors,
- Fig. 3: eine der Fig. 2 entsprechende Darstellung im belasteten Zustand des Drucksensors,
- Fig. 4: eine Draufsicht der Vorrichtung,
- Fig. 5: die Klickfeder in einer Seitenansicht und einer Draufsicht und
- Fig. 6: eine Schnittansicht der Vorrichtung gemäß einer zweiten Ausführungsform

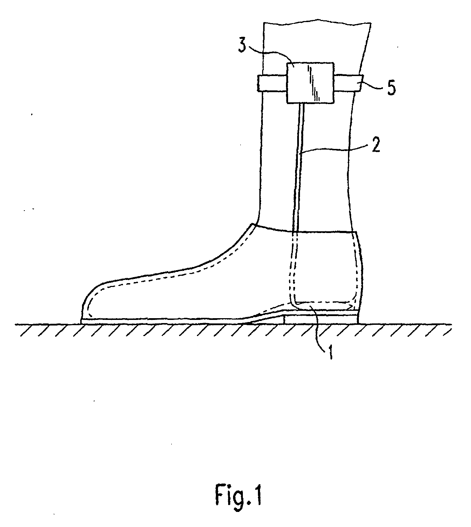

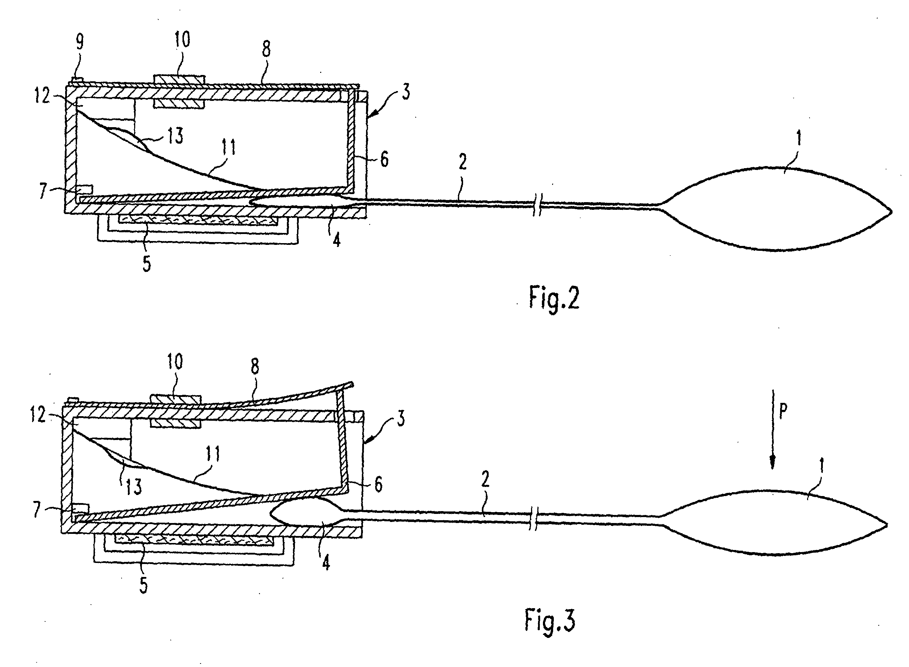

Ausweislich der Fig. 1, welche die Gesamtheit der Vorrichtung zur Lieferung eines akustischen Signals bei der Erfassung des auf einem Fuß lastenden Körpergewichts eines Patienten bspw. im Rahmen der Therapie einer Beinfraktur zeigt, besteht die Vorrichtung aus einem Fußkissen 1, das als ein Drucksensor für eine Anordnung unter der Ferse des Beines vorgesehen ist, welches bei dem Patienten zur Verhinderung der Überschreitung einer vorbestimmten Belastungsgrenze zu überwachen ist. Der Drucksensor 1 ist dafür als eine mit einem Fluid gefüllte Blase aus einem elastisch deformierbaren Material ausgebildet und ist durch ein Schlauchstück 2 mit einem in einem Gehäuse 3 untergebrachten Druckwächter 4 verbunden, der ausweislich der Fig. 2 bis 4 ebenfalls als eine Blase aus einem elastisch deformierbaren Material ausgebildet ist. Das Gehäuse 3 ist bspw. für eine Befestigung mittels eines Verschlußbandes 5 an dem Bein des Patienten vorgesehen. Die beiden Blasen 1 und 4 sowie das verbindende Schlauchstück 2 sind bspw. mit zwei übereinander gelegten und miteinander verschweißten Kunststoffolien gebildet und sind mit einer vorbestimmten Wassermenge gefüllt, sodaß damit eine autonomer, separat auswechselbarer Körper zur Verfügung steht, mit welchem das auf dem Fuß des Patienten lastende Körpergewicht erfaßt werden kann. Die als ein Drucksensor dienende eine Blase 1 ist dabei als ein Fußkissen für eine Anordnung bspw. unter der Ferse des Patienten vorgesehen, jedoch kann sie gleichermaßen für eine Anordnung unterhalb des Fußballens benutzt werden, sofern die Therapie einer Beinfraktur oder einer anderen orthopädischen Operation bspw. der Hüfte eine diesbezügliche Erfassung der Belastung mit dem Körpergewicht dem behandelnden Arzt sachdienlicher erscheinen sollte.

Mit der innerhalb des Gehäuses 3 als ein Druckwächter angeordneten Blase 4 ist ein L-förmiger Betätigungshebel 6 in Berührung gehalten. Der Betätigungshebel 6 ist an seinem einen Schenkel durch ein Widerlager 7 gegen den Boden des Gehäuses abgestützt und steht mit seinem anderen Schenkel an einer Gehäuseöffnung über die Gehäusedecke vor, sodaß dieser Schenkel an seinem freien Ende mit einer Blattfeder 8 belastet werden kann, die an ihrem einen Ende bei 9 an dem Gehäuse 3 eingespannt ist. Diese Blattfeder 8 dient als eine Rückstellfeder, welche den Betätigungshebel 6 gegen den Druckwächter 4 vorspannt, wobei diese Vorspannung durch das Widerlager 7 aufgefangen wird.

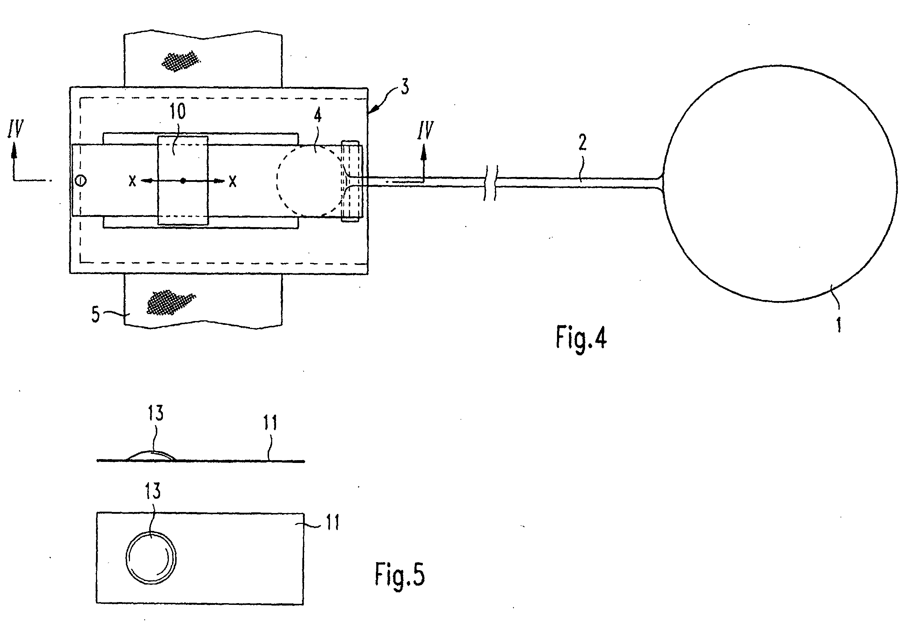

Mit dem Hebel 6 wird der Fluiddruck abgetastet, der in der als ein Druckwächter dienenden Blase 4 vorherrscht und über das Schlauchstück 2 von dem als Drucksensor dienenden Fußkissen in Abhängigkeit davon vermittelt wird, welche Gewichtsbelastung die Blase 1 erfährt. Die Gewichtsbelastung der Blase 1 führt zu einer Verdrängung des in ihr gespeicherten Wassers hin zu der Blase 4, sodaß sich diese aus dem nahezu flach gelegten Zustand der Fig. 2 in einen aufgeblähten Zustand verformt, wie es in Fig. 3 veranschaulicht ist. Durch diese Verformung der Blase 4 wird der Hebel 6 entgegen der Vorspannkraft der Feder 8 um das Widerlager 7 verschwenkt, wobei mit einem Schieberglied 10 der Härtegrad der Blattfeder 8 auf einen vorbestimmten Wert eingestellt werden kann, der bspw. einer Belastungsgrenze von 20 kg für den auf dem Fußkissen der Vorrichtung lastenden Fuß des Patienten entspricht. Durch ein Verschieben des Schiebergliedes 10 in der in Fig. 4 mit dem Doppelpfeil x-x angedeuteten Richtung kann der Härtegrad der Blattfeder 8 auf einen niedrigeren oder höheren Wert verstellt werden, sofern das Schieberglied nach links oder nach rechts verstellt wird.

Der von dem Betätigungshebel 6 abgetastete Fluiddruck, der also in der Blase 4 in Abhängigkeit von der Druckbelastung der Blase 1 unter Vermittlung des Schlauchstückes 2 vorherrscht, wird zur Lieferung eines akustischen Signals durch einen Signalgeber übersetzt, sobald dieser Fluiddruck eine Größe erreicht hat, die einer vorbestimmten Belastungsgrenze des Fußes des Patienten entspricht. Der Signalgeber ist als eine sog. Klickfeder 11 ausgebildet, die innerhalb des Gehäuses 3 mit ihrem einen Ende an einem Widerlager 12 eingespannt ist und mit ihrem anderen Ende an dem einen Schenkel des Betätigungshebels 6 anliegt. Diese Klickfeder 11 ist mit einer Auswölbung 13 versehen, die bei einer durch den Betätigungshebel 6 beim Erreichen eines vorbestimmten Fluiddruckes in der Blase 4 in Abhängigkeit von der mit dem Härtegrad der Blattfeder 8 vorbestimmten Belastungsgrenze ausgelösten Betätigung eine Rückformung erfährt und dabei ein hörbares Klickgeräusch erzeugt. Die Rückformung der Auswölbung 13 ist in Fig. 3 verdeutlicht, wobei die im Vergleich zu der Darstellung in Fig. 2 gezeigte Zustandsänderung der Auswölbung so zu verstehen ist, daß bei dieser Zustandsänderung das hörbare Klickgeräusch ebenso erzeugt wird wie es nochmals zu vernehmen ist, wenn sich die Auswölbung 13 von dem Zustand der Fig. 3 wieder in den Zustand der Fig. 2 verändert. Dieses von der Klickfeder 11 abgegebene hörbare Klickgeräusch erfährt dabei eine Verstärkung durch das als ein Resonanzkasten wirkende Gehäuse 3, und es ist somit dieses Klickgeräusch, welches dem Patienten vermittelt, daß er bei der mit der Vorrichtung überwachten Druckbelastung des Fußes mit dem Körpergewicht eine vorbestimmte Belastungsgrenze überschritten hat bzw. deren Unterschreitung wieder erhalten ist, sobald sich das Klickgeräusch wiederholt hat.

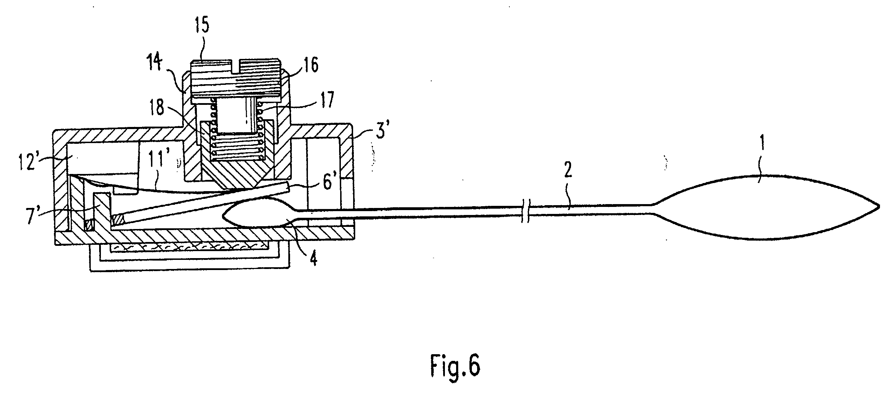

Bei der in Fig. 6 gezeigten alternativen Ausführungsform der Vorrichtung ist als Ersatz für den L-förmigen Hebel 6 eine Druckplatte 6' vorgesehen, die an einem bodenseitigen Vorsprung 7' des Gehäuses 3' schwenkbar gelagert ist. Die Druckplatte 6' ist auch hier mit der Blase 4 des Druckwächters unterlegt und dient der Abstützung des einen Endes der Klickfeder 11', deren anderes Ende durch ein Widerlager 12' abgestützt ist.

Abweichend von der vorbeschriebenen ersten Ausfünrungsform der Vorrichtung ist das Gehäuse 3' mit einer Führungsbuchse 14 ausgebildet, in welcher eine Stellschraube 15 über ein zusammenwirkendes Gewinde 16 verschraubt ist. An der Stellschraube 15 ist eine Druckfeder 17 abgestützt, die einen in der Führungsbuchse 14 geführten Taster 18 gegen die Klickfeder 11' vorspannt. Die Vorspannung der Druckfeder 17 kann mittels der Stellschraube 15 verändert werden, sodaß damit ähnlich wie mit dem Schieberglied 10 auch bei dieser Ausführungsform die für den Fuß des Patienten überwachte Belastungsgrenze auf jeden gewünschten Wert eingestellt werden kann, bei dessen Erreichen dann die Klickfeder 11' für die Abgabe eines Klickgeräusches aus ihrer Ruheposition heraus bewegt wird.

## Patentansprüche

1. Vorrichtung zur Lieferung eines akustischen Signals bei der Erfassung des auf einem Fuß lastenden Körpergewichts in Abhängigkeit von der Druckbelastung eines mit einem Fluid gefüllten, elastisch deformierbaren Fußkissens, das als ein Drucksensor für das Körpergewicht über eine Fluidleitung mit einem Druckwächter verbunden ist, der zur Betätigung eines Signalgebers in Abhängigkeit von dem über die Fluidleitung vermittelten Fluiddruck vorgesehen ist,
**dadurch gekennzeichnet,**
**daß** der Signalgeber mit einer durch den Druckwächter (4) zur Abgabe eines hörbaren Klickgeräusches betätigbaren Klickfeder (11, 13) ausgebildet ist.

2. Vorrichtung nach Anspruch 1, **dadurch gekennzeichnet, daß** die Klickfeder (11, 13) in einem zur Verstärkung des Klickgeräusches als eine Art Resonanzkörper vorgesehenen Gehäuse (3) angeordnet ist.

3. Vorrichtung nach Anspruch 1 oder 2, **dadurch gekennzeichnet, daß** die Klickfeder (11, 13) gegen einen durch den Druckwächter (4) betätigbaren Betätigungshebel (6) verspannt ist, der in Abhängigkeit von dem an dem Druckwächter abgetasteten Fluiddruck die Klickfeder für die Abgabe des Klickgeräusches aus der Ruhestellung heraus bewegen kann.

4. Vorrichtung nach einem der Ansprüche 1 bis 3, **dadurch gekennzeichnet, daß** die Klickfeder (11, 13) in ihre Ruhestellung durch eine Rückstellfeder (8, 17) vorgespannt ist.

5. Vorrichtung nach einem der Ansprüche 1 bis 4, **dadurch gekennzeichnet, daß** die Rückstellfeder mit einer an dem Gehäuse (3) eingespannten Blattfeder (8) gebildet ist, deren Härtegrad für eine Veränderung der auf die Klickfeder (11, 13) einwirkenden Vorspannkraft durch ein Schieberglied (10) des Gehäuses (3) verstellbar ist.

6. Vorrichtung nach einem der Ansprüche 1 bis 4, **dadurch gekennzeichnet, daß** die Rückstellfeder mit einer Druckfeder (17) gebildet ist, die für die Ausübung einer veränderlichen Vorspannkraft auf die Klickfeder (11') an einer mit dem Gehäuse (3') verschraubten Stellschraube (15) abgestützt ist.

7. Vorrichtung nach einem der Ansprüche 1 bis 6, **dadurch gekennzeichnet, daß** das Fußkissen (1) und der Druckwächter (4) jeweils mit einer Blase aus einem elastisch deformierbaren Material ausgebildet und zur Bereitstellung eines autonomen, separat auswechselbaren Körpers durch ein Schlauchstück (2) miteinander verbunden und mit einem Fluid in solcher Menge gefüllt sind, daß damit ein vorbestimmter Bereich von Belastungsgrenzen überwacht werden kann.

## Claims

1. A device for providing an acoustic signal to a patient when a predetermined load is placed on the patient's foot comprising:
a resilient foot pad filled with a fluid and serving as a pressure sensor for the patient's weight for sensing the load exerted via the patient's foot to the foot pad; and
a fluid line connecting the resilient foot pad to a pressure responsive means communication with the liquid and adapted to actuate a signaling means at a predetermined pressure of the liquid corresponding to a predeterminded load when placed on the patient's foot;
wherein said signaling means comprises a click spring (11, 13) having a deformable bulge (3) and being resiliently supported such as to supply an audible click noise when actuated by said pressure responsive means whereby the click noise is obtained by a transient deformation of the bulge whenever the click spring is leaving its rest position and is again returning to the same.

2. The device of claim 1 in which the click spring (11, 13) is arranged within a box (3) serving as a resonance body for amplifying its click noise.

3. The device of claim 1 in which the click spring (11, 13) is biased against a lever (6) which is in operational contact with the pressure responsive means and adapted to actuate the click spring for supplying its click noise.

4. The device of claim 1 - 3 in which the click spring (11, 13) is biased by a pull-back spring (8, 17).

5. The device of claim 1 - 4 in which the click spring is biased by a leaf spring (8) which is fixed to the box (3) in such a manner that its biasing force acting via a lever against the pressure responsive means may be adjustied by a slide member to thereby adjust the predetermined load at which the click spring (11, 13) will supply its click noise.

6. The device of claim 1 - 4 in which the click spring is biased by a compression coil spring (17) acting between a feeler which is in contract with the click spring (11') and an adjustible set screw (15).

7. The device of claim 1 - 6 in which the resilient foot pad and the pressure responsive means are both provided by a bladder (1) of a resilient material and are interconnected by a flexible tube (2) to thereby obtain an autonomous body (4) filled with fluid as a sparate exchangeable control means for actuating the click spring.

## Revendications

1. Dispositif qui envoie un signal acoustique lors de la détection du poids corporel reposant sur le pied. La charge de pression est reflétée sur un coussin élastique déformable, rempli d'un fluide, c'est -à dire un détecteur de pression du poids corporel relié par une conduite de fluide à un manostat. La pression du fluide est transmise par la conduite et transmet un signal.
Caractéristiques :
le transmetteur de signal est sollicité par le manostat et émet un déclic audible provoqué par des ressorts à déclic.

2. Le dispositif après sollicitation décrite en point 1 est **caractérisé par le fait que** les ressorts à déclic (11, 13) sont placés sur une sorte de boîtier servant de boîte de résonance pour intensifier le bruit du déclic.

3. Le dispositif après sollicitation décrite en point 1 ou 2 est **caractérisé par le fait que** les ressorts à déclic (11 et 13) se trouvent tendus contre un levier (6) activé par le manostat qui ayant déterminé la pression du fluide déclanche l'émission du bruit de déclic.

4. Le dispositif après sollicitation 1 - 3 est **caractérisé par le fait que** les ressorts à déclic (11, 13) sont maintenus en pré-tension en position neutre par un ressort de rappel.

5. Le dispositif après sollicitation 1 - 4 se **caractérise par le fait que** le ressort de rappel est formé d'un ressort à lames (8) encastré dans le boîtier. Sa dureté est réglable à l'aide d'un poussoir ajusté sur le boîtier. Ce ressort à lames influence la pré-tension des ressorts à déclic.

6. Le dispositif après sollicitation 1 - 4 est **caractérisé par le fait que** le ressort de rappel est formé d'un ressort pression qui, pour exercer une force de pré-tension variable sur les ressorts à déclic, s'appuie sur une vis de réglage vissée au boîtier.

7. Le dispositif après sollicitation 1 - 6 est **caractérisé par le fait que** le coussin (1) et le manostat (4) sont respectivement formés d'une vessie conçue en un matériau élastique déformable. Pour la mise à disponibilité d'un corps autonome, et amovible séparément, ils sont reliés par un morceau de conduite (2) remplie d'un fluide en quantité appropriée pour contrôler une plage déterminée de charges limite.
